# EUROPEAN PATENT SPECIFICATION

(11) **EP 3 031 399 B1**
(45) Date of publication and mention of the grant of the patent: **17.02.2021**
(21) Application number: 15198054.7
(22) Date of filing: 04.12.2015
(51) Int. Cl.: A61B 8/10, A61B 8/08

(54) **EQUIPMENT AND METHOD FOR ULTRASOUND IMAGING OF AN EYE**
AUSRÜSTUNG UND VERFAHREN ZUR ULTRASCHALLABBILDUNG EINES AUGES
ÉQUIPEMENT ET PROCÉDÉ D'IMAGERIE PAR ULTRASONS D'UN OEIL

(30) Priority: 09.12.2014 IT CO20140039
(43) Date of publication of application: 15.06.2016
(73) Proprietor: Nidek Co., Ltd., Gamagori-Aichi, 443-0038 (JP)
(72) Inventor: TANASSI, Cesare, 35020 Albignasego (Padova) (IT); BUSCEMI, Phil, 35020 Albignasego (Padova) (IT); BISSON, Raffaella, 35020 Albignasego (Padova) (IT); PASCOLINI, Michele, 35020 Albignasego (Padova) (IT); CODOGNO, Nicola, 35020 Albignasego (Padova) (IT); PAJARO, Simone, 35020 Albignasego (Padova) (IT)
(74) Representative: De Ros, Alberto

(56) References cited:
- WO-A1-2015/070889
- JP-A- S62 106 749
- US-A- 4 823 801
- US-A1- 2008 200 807
- US-A1- 2012 089 021
- US-A1- 2014 249 422

## Description

### TECHNICAL FIELD

The present invention relates to an equipment and a method for ultrasound imaging of an eye.

### BACKGROUND ART

Automatic ultrasound imaging of eyes for examination or therapeutic purposes is not widespread, i.e. there are few equipments available on the market.

The Applicant is aware of an ultrasound equipment available on the market that is known under the commercial name "ArcScan").

Japanese patent application, published as "JPS62106749A", and US 4,823,801 disclose a cornea thickness measuring ultrasonic probe. The thickness is measured by means of ultrasonic waves generated by a plurality of ultrasonic wave vibrators disposed on at least one meridian. The main measurement is in the center of the cornea; other measurements are at different points of the cornea.

US patent application, published as "US2012089021A1", discloses a method and a system for three-dimensional imaging of biological structures, such as eyes. The ultrasonic scanning apparatus includes a unique ultrasonic array that slides back and forth along a linear translation rod during examination; the distance between array and the eye continuously varies from e.g. few centimeters to e.g. few millimeters.

International patent application, published as "WO2015070889A1" and falling under Article 54(3) EPC, discloses a device and a method for performing thermal keratoplasty using high intensity focused ultrasounds. The device comprises a plurality of ultrasonic transducers for emitting ultrasound waves; the ultrasound waves of some transducers are focused on corresponding areas of the cornea in order to heat these areas and cause collagen shrinkage; some (preferably all) transducers are capable of receiving ultrasound waves for ocular imaging of the areas of the cornea under treatment. The transducers may be arranged in at least one ring array.

These and other conventional ultrasound equipments suffer drawbacks. For example, they comprise many moving parts that must move with high precision and accuracy with respect to the eye and, as a result, their design is very complicated due to the requisite high precision and accuracy. Further, during operation of the equipment, it is inconvenient for the patient that has to dip his eye in a small tub full of liquid in order for the devices to function properly.

These and other drawbacks are addressed by the present invention.

### SUMMARY

It is the general task of the present invention to improve the technology of the eye ultrasound imaging and to provide a more simple equipment for ultrasound imaging a plurality of cross-sectional areas of a peripheral annular volume of an eye; preferably, such volume should include the iridocorneal part of the eye and also all the structures that are located behind the trabeculate and iris.

The basic idea behind the present invention is to leverage on the flexibility of solid-state ultrasound emitting and receiving devices, in particular cMUTs. Thanks to their reduced dimensions, an array of devices may be arranged for example in an annular manner and positioned close to the eye scleral limbus in a stationary manner. In this way, cross-sectional images sections may be acquired quickly and easily.

The present invention is defined by the appended claims.

A first aspect the present invention corresponds to an equipment for ultrasound imaging a plurality of cross-sectional areas of a peripheral annular volume of an eye; it comprises :
- a mechanic device having a front surface designed to be located close to a front surface of a cornea of the eye, and
- a plurality of ultrasound emitting and receiving electronic devices associated with said mechanic device, arranged in an annular manner so as to be distributed around the eye when
said mechanic device is located close to the front surface of the cornea of the eye, each of said electronic devices being directed toward a cross-sectional area of the peripheral annular volume of the eye opposite to the device with respect to the eye axis;
said mechanic device is designed to be stationary with respect to the eye;
said electronic devices are designed to be stationary with respect to the eye.

The equipment may advantageously further comprise another device; the other device and the mechanic device are fixed to each other (i.e. they do not move with respect to each other); the electronic devices are fixed to the mechanic device (i.e. they do not move with respect to the mechanic device).

According to some embodiments, the other device serves only as a disposable device. According to some embodiments, the other device serves only as an optical device for the optical observation of an iridocorneal annular zone of the eye.

According to some embodiments, the other device serves both as a disposable device and as an optical device.

According to some embodiments, the mechanic device serves also as an optical device for the optical observation of an iridocorneal annular zone of the eye.

A second aspect of the present invention corresponds to method of ultrasound imaging a peripheral annular volume of an eye through a mechanic device having a concave front surface and a plurality of ultrasound emitting and receiving electronic devices associated with the device and arranged in an annular manner; it comprises the steps of :
A) applying a viscous transparent coupling agent to a front surface of a cornea of the eye and/or to a concave front surface of the mechanic device,
B) positioning the mechanic device so that the front surface of the mechanic device is close to the front surface of the eye and so that said electronic devices are distributed around the eye and each of said devices is directed toward a cross-sectional area of the peripheral annular volume of the eye opposite to the device with respect to the eye axis,
C) detecting a plurality of cross-sectional areas of the peripheral annular volume of the eye through the plurality of ultrasound emitting and receiving electronic devices;
during step C said mechanic device and the electronic devices are maintained stationary with respect to the eye.

According to some embodiments, the plurality of cross-sectional areas are detected contemporaneously or almost contemporaneously; the various detecting actions are typically independent from each other.

According to some embodiments, the method comprises further the step of disposing the mechanic device after step C.

According to some embodiments, the mechanic device is transparent only to ultrasounds or only to (visible) light or to both ultrasounds and (visible) light.

According to some embodiments, both ultrasound imaging and optical observation are carried out

The invention also provides a method as defined in claim 15. Preferred embodiments are defined in the dependent claims.

Furthermore, treatment of tissues in the peripheral annular volume of the eye by radiating them with ultrasounds may be provided, but does not form part of the present invention.

### BRIEF DESCRIPTION OF DRAWINGS

The accompanying drawings, which constitute a part of the specification, illustrate exemplary embodiments of the present invention and, together with the detailed description, explain these embodiments. In the drawings:
Fig.1A shows a schematic cross-section view of a human eye,
Fig.1B shows a schematic front view of a human eye together with a plurality of cross-sectional areas A and a corresponding plurality of ultrasound devices G,
Fig.2A shows a schematic cross-section view of a human eye wherein two cross-sectional areas are highlighted,
Fig.2B shows an enlarged view of one of the cross-sectional areas of Fig.2A,
Fig.3 shows a schematic cross-section view of a first embodiment an equipment according to the present invention,
Fig.4 shows a schematic cross-section view of a second embodiment an equipment according to the present invention,
Fig.5 shows a schematic tridimensional partial view of the equipment of Fig.4,
Fig.6 shows a schematic cross-section view of a third embodiment an equipment according to the present invention,
Fig.7 shows a schematic tridimensional partial view of the equipment of Fig.6,
Fig.8 shows a schematic cross-section view of a fourth embodiment an equipment according to the present invention, and
Fig.9 shows a schematic cross-section view of a fifth embodiment an equipment according to the present invention.

### DETAILED DESCRIPTION

The following detailed description of exemplary embodiments refers to the accompanying drawings.

The following detailed description does not limit the present invention. Instead, the scope of the present invention is defined by the appended claims.

Reference throughout the specification to "one embodiment" or "an embodiment" means that a particular feature, structure, or characteristic described in connection with an embodiment is included in at least one embodiment of the subject matter disclosed. Thus, the appearance of the phrases "in one embodiment" or "in an embodiment" in various places throughout the specification is not necessarily referring to the same embodiment.

In the following, the expression "a mechanic device" will be used that may also be replaced by "a first mechanic device" or "a main mechanic device", and the expression "another mechanic device" will be used that may also be replaced by "a second mechanic device" or "an additional mechanic device".

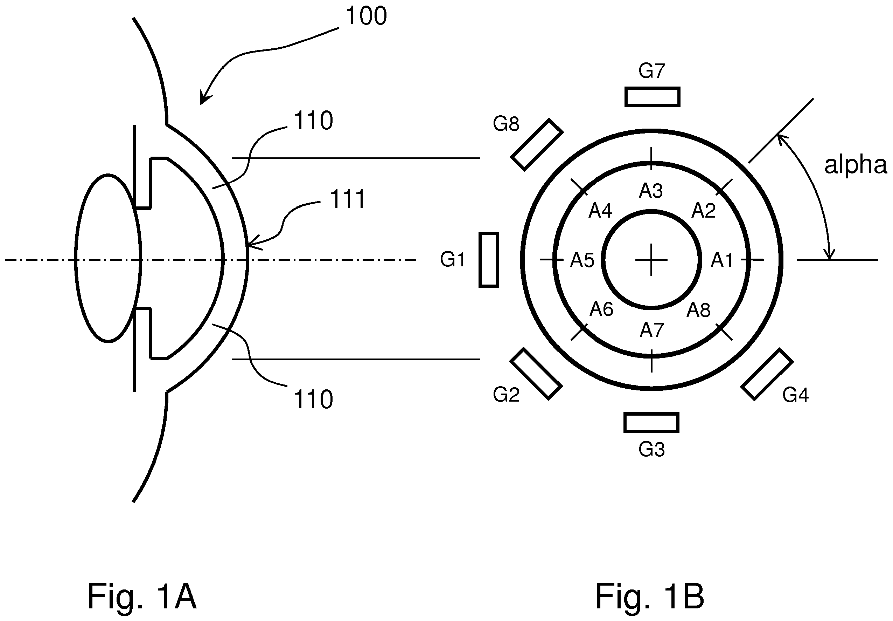

Fig.1A shows a schematic cross-section view of a human eye 100; reference 110 corresponds to the cornea and reference 111 corresponds to the front surface of the cornea.

Fig.1B shows a schematic front view of a human eye 100 together with a plurality of cross-sectional areas A1, A2, A3, A4, A5, A6, A7, A8 and a corresponding plurality of ultrasound emitting and receiving electronic devices G1, G2, G3, G4, G5, G6, G7, G8; device G1 (or a group of devices G1) is opposite to area A1 (with respect to the eye axis) and used for imaging area A1, device G2 (or a group of devices G2) is opposite to area A2 (with respect to the eye axis) and used for imaging area A2, etc. ; the areas A and the devices G are regularly distributed around the eye and the angle between two adjacent ones is "alpha" (that in particular is equal to 45°). This figure shows eight areas and eight devices, but according to the present invention a different number may be provided; typically, the minimum number is four and the maximum number is sixteen.

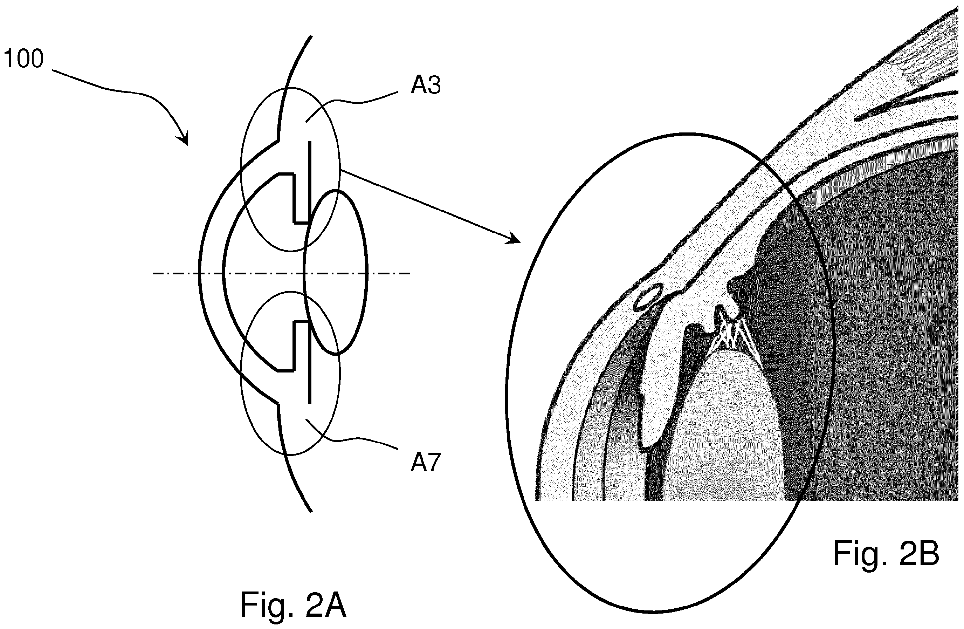

Fig.2A shows a schematic cross-section view of the human eye 100 wherein two cross-sectional areas A3 and A7 are highlighted.

Fig.2B shows an enlarged view of the cross-sectional area A3 of Fig.2A. According to the present invention, the structures (including tissues) that should be imaged through ultrasound are preferably cornea (and all its constituents), conjunctiva, angle of anterior chamber, trabecular meshwork, Schlemm's canal, scleral spur, iris, posterior chamber of the eye, ciliary body, Zinn's zonules, crystalline lens; these structures are approximately contained in the ellipse of Fig.2B.

The present invention aims at ultrasound imaging a peripheral annular volume of the anterior part of the eye, typically an internal volume. Preferably, an angle of at least 90° is covered through a single imaging process; more preferably the angle is at least 180°; even more preferably the angle is 360°.

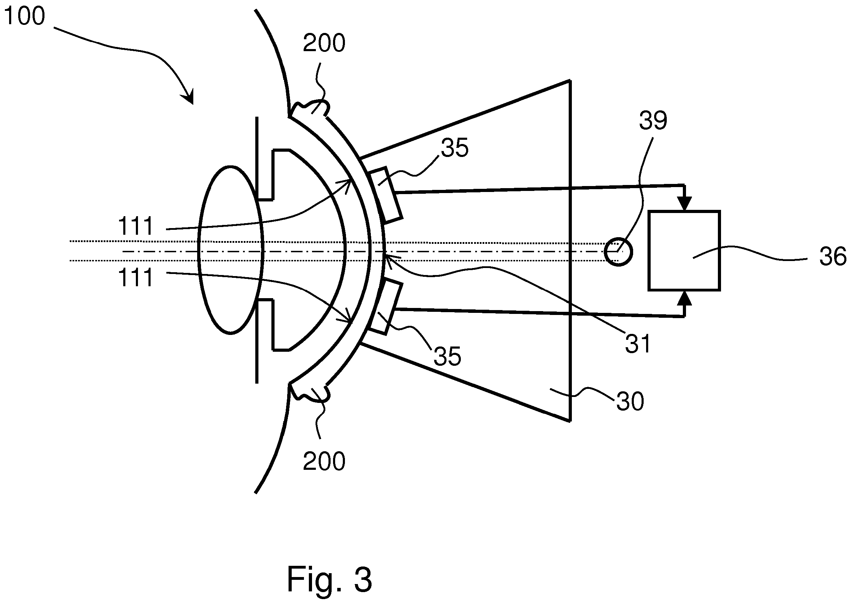

To this aim, there are used a mechanic device (reference 30 in Fig.3) having a front surface (reference 31 in Fig.3) and a plurality of ultrasound emitting and receiving electronic devices (reference 35 in Fig.3 and references G1, G2, G3, G4, G5, G6, G7, G8 in Fig.1B) associated with the mechanic device (reference 30 in Fig.3) arranged in an annular manner; the electronic devices may be integrated in the mechanic device or mounted to the mechanic device so that the mechanic device is a support for the electronic devices (alternatively, undirect support may be provided by the mechanic device).

In general, the method comprises the steps of :
A) applying a viscous transparent coupling agent (reference 200 in Fig.3) between a front surface (reference 111 in Fig.3) of a cornea of the eye (reference 100 in Fig.3) and the front surface (reference 31 in Fig.3) of the mechanic device (reference 30 in Fig.3),
B) positioning the mechanic device (reference 30 in Fig.3) so that the front surface (reference 31 in Fig.3) of the mechanic device (reference 30 in Fig.3) is close or proximal the front surface (reference 111 in Fig.3) of the eye and so that the electronic devices are distributed around the eye (100) when said mechanic device (30) is located close or proximal to the front surface (111) of the cornea (110) of the eye (100), each of said electronic devices being directed toward a cross-sectional area (A1, A2, A3, A4, A5, A6, A7, A8) of the peripheral annular volume of the eye (100),
C) detecting a plurality of cross-sectional areas (references A1, A2, A3, A4, A5, A6, A7, A8 in Fig.1B) of the peripheral annular volume of the eye through the plurality of ultrasound emitting and receiving electronic devices (reference 35 in Fig.3 and references G1, G2, G3, G4, G5, G6, G7, G8 in Fig.1B);
during step C, the mechanic device (reference 30 in Fig.3) is maintained stationary with respect to the eye and the electronic devices (reference 35 in Fig.3 and references G1, G2, G3, G4, G5, G6, G7, G8 in Fig.1B) are maintained stationary with respect to the eye.

According to a good practice, the distance between the mechanic device, specifically the front surface of the mechanic device, and the cornea should be the range 0.5-2.5 mm, preferably in the range 1.0-2.0 mm; shorter distances and longer distances should be avoided; in particular, contact between the mechanic device and the eye should be avoided and a layer of a viscous transparent coupling agent should be between them.

Viscous transparent (to both light and ultrasounds) coupling agent are already available on the market; for example ophthalmic gels are used for gonioscopic examinations.

Several equipments allow to implement such general method; in the following five different embodiments will be described.

Preferably, each of the plurality of cross-sectional areas A1, A2, A3, A4, A5, A6, A7, A8 is detected through a distinct group of electronic devices G1, G2, G3, G4, G5, G6, G7, G8.

Fig.3 shows a first embodiment.

According to this embodiment, there are:
- a solid mechanic device 30 having a concave front surface 31 designed to be located close or proximal to a front surface 111 of a cornea an eye, in particular approximately matching the convex front surface 111 of the cornea of the eye, and
- a plurality of ultrasound emitting and receiving electronic devices 35 fixed to the mechanic device 30;
the mechanic device 30 is designed to be stationary with respect to the eye (i.e. the equipment does not comprise any actuator for device 30);
the electronic devices 35 are designed to be stationary with respect to the eye (i.e. the equipment does not comprise any actuator for devices 35).

The mechanic device 30 has approximately the shape of a cylinder or a prism or a truncated cone or truncated pyramid, or is annular.

The electronic devices 35 are cMUT devices.

Additionally, there is analog and/or digital signal processing circuitry 36 located close to the devices 35, for example outside the mechanic device 30 but adjacent thereto.

Finally, there is a "fixation target" 39 for maintaining the eye in a fixed and predetermined position during ultrasound imaging; the so-called "fixation target" is usually a small luminous device that attracts and maintain the attention of an eye; centrally, the mechanic device 30 allows the propagation of the light beam of the "fixation target" 39 either because it is transparent to light or because it has a hole.

As each of the elements G1, G2, G3, G4, G5, G6, G7, G8 may correspond to a group of ultrasound electronic devices, the group may be integrated in a microelectronic chip. Some analog and/or digital signal processing circuitry may be located in said chip and/or some analog and/or digital signal processing circuitry may be located close to said chip and/or some analog and/or digital signal processing circuitry may be located remote from said chip.

The plurality of cross-sectional areas (for example A1, A2, A3, A4, A5, A6, A7, A8) detected by devices 35 cover the whole peripheral annular internal volume of the eye.

According to a variant of the embodiment of Fig.3, the mechanic device may be also an optical device for the optical observation of an iridocorneal annular zone of the eye. In this case, the mechanic device 30 should allow propagation of light and the electronic devices 35 should not impede propagation of imaging light beams.

It is to be noted that, very advantageously, the electronic devices 35 may be arranged not only for imaging but also to treat tissues in the peripheral annular volume of the eye by radiating them with ultrasounds.

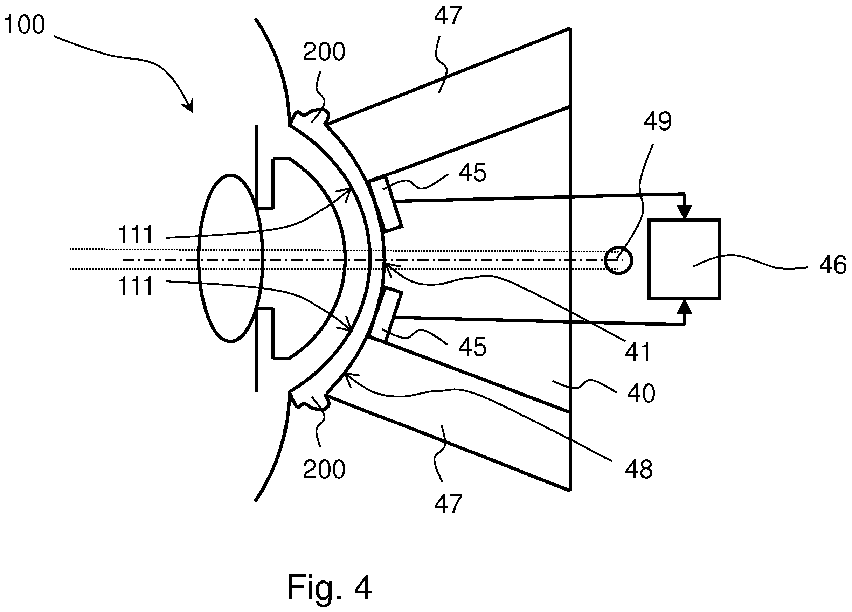

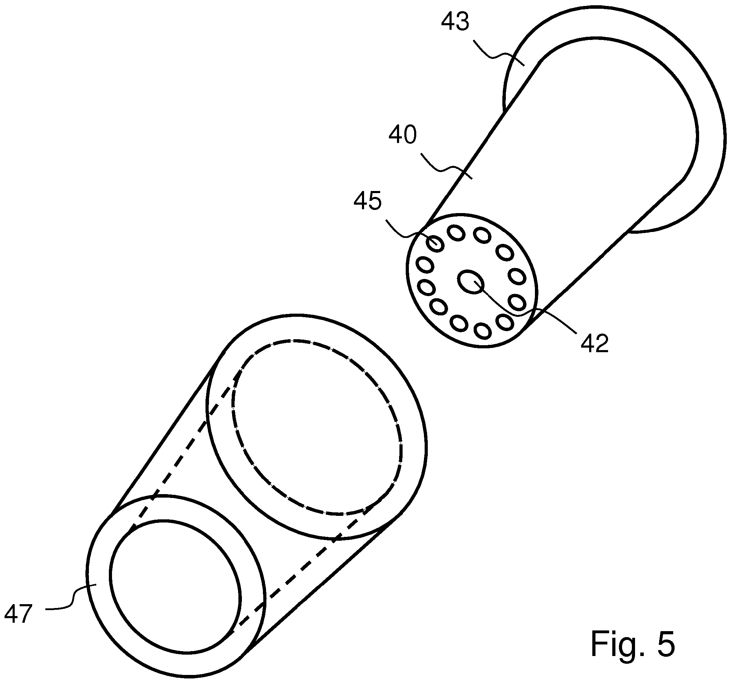

Fig.4 and Fig.5 show a second embodiment.

This embodiment is similar to the embodiment of Fig.3 but it comprises further another device 47. The other device 47 and the mechanic device 40 are fixed (integrated or mounted) to each other; in particular, the other device 47 is fixed (integrated or mounted) to the mechanic device 40 and the mechanic device 40 is inserted into a through hole of the other device 47 so that the other device 47 is located around the mechanic device 40.

Fig.5 shows a hole 42 for the light beam of the "fixation target" 49 and a rim 43 for fixing (e.g. mounting) the devices together.

The other device 47 is an optical device for the optical observation of an iridocorneal annular zone of the eye.

The front surface 41 of device 40 and the front surface 48 of device 47 are (substantially) aligned on a common curved surface.

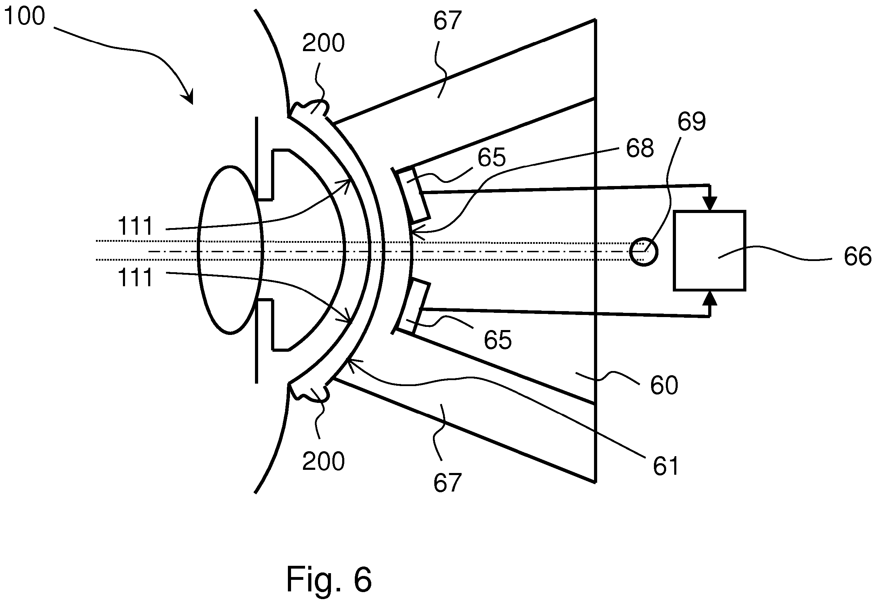

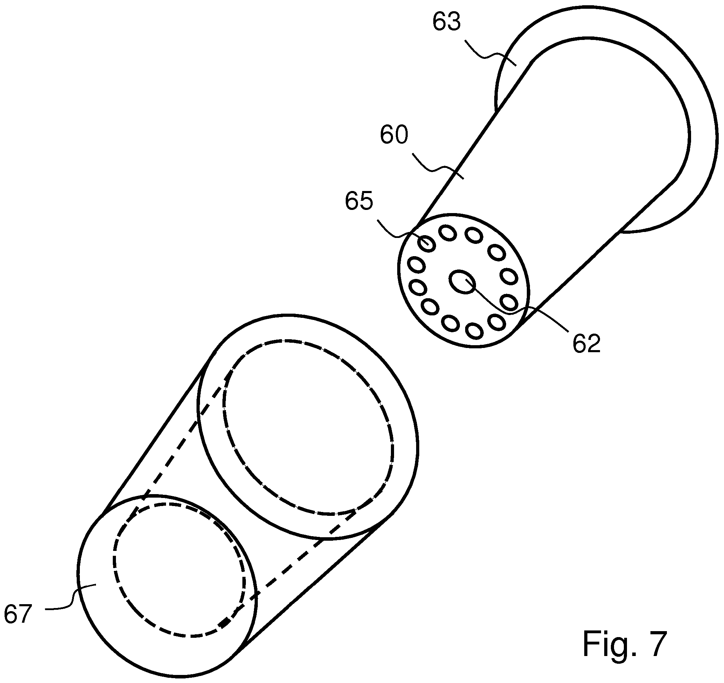

Fig.6 and Fig.7 show a third embodiment.

This embodiment is similar to the embodiment of Fig.4 and Fig.5 but the hole of the other device 67 is a blind hole wherein the mechanic device 60 is located after been inserted so that the other device 67 is located around the mechanic device 60.

According to this embodiment, the concave front surface 61 of the other device 67 is very close to the front surface 111 of the cornea of the eye and the front surface 68 of the mechanic device 60 is less close to the front surface 111 of the cornea of the eye being behind the front surface 61; preferably, the concave front surface 61 matches the convex front surface 111 of the cornea of the eye; preferably, the front surface 68 is also concave.

According to this embodiment, at least the front portion of the other device 67 should be of a material transparent to both light and ultrasounds; a material that is sufficiently transparent to visible light and that allows good transmission of ultrasound waves is PMMA [poly(methylmethacrylate)].

According to this embodiment, the other device 67 may be disposable and the mechanic device 60 may be permanent as it does not contact the eye, in particular the cornea at all.

Fig.7 shows a hole 62 for the light beam of the "fixation target" 69 and a rim 63 for fixing (e.g. mounting) the devices together.

The other device 67 is an optical device for the optical observation of an iridocorneal annular zone of the eye.

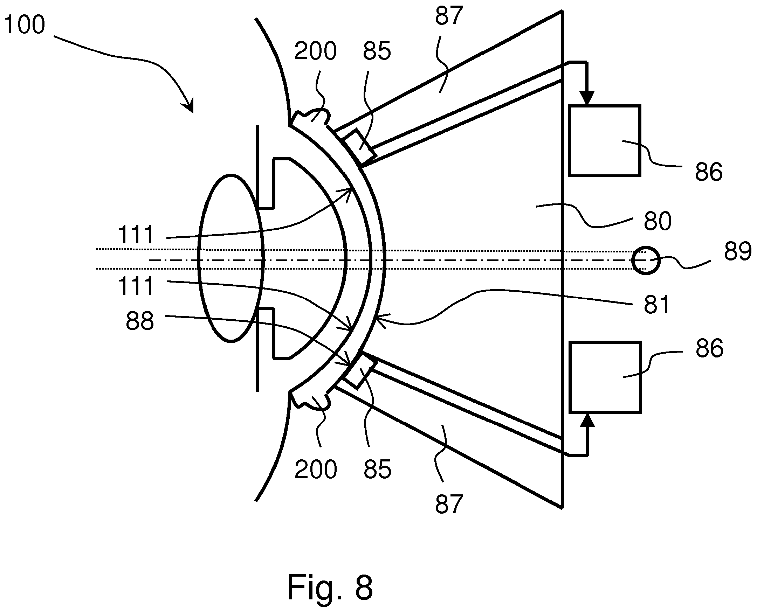

Fig.8 shows a fourth embodiment.

The embodiment of Fig.8 is similar to embodiment of Fig.4 but the mechanic device 87 is located around the other device 80, i.e. the optical device. The other device 87 and the mechanic device 80 are fixed (integrated or mounted) to each other; in particular, the mechanic device 87 is fixed (integrated or mounted) to the other device 80 and the other device 80 is inserted into a through hole of the mechanic device 87.

The front surface 81 of device 80 and the front surface 88 of device 87 are (substantially) aligned on a common curved surface.

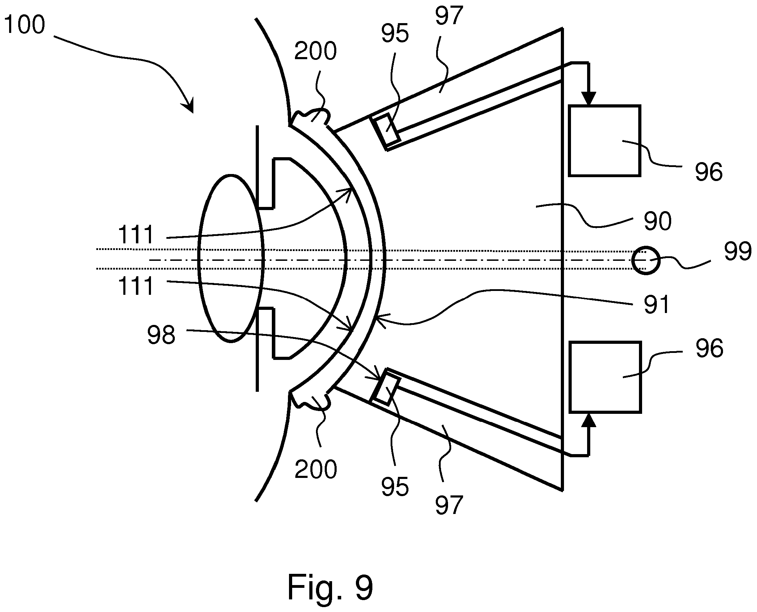

Fig.9 shows a fifth embodiment.

The embodiment of Fig.9 is similar to embodiment of Fig.6 but the mechanic device 97 is located around the other device 90, i.e. the optical device; in particular, device 97 is fixed (integrated or mounted) to the device 90 in a lateral recess of device 90.

According to this embodiment, the concave front surface 91 of the other device 90 is very close to the front surface 111 of the cornea of the eye and the front surface 98 of the mechanic device 97 is less close to the front surface 111 of the cornea of the eye being behind the front surface 91; preferably, the concave front surface 91 matches the convex front surface 111 of the cornea of the eye; preferably, the front surface 98 is also concave.

According to this embodiment, at least the front portion of the other device 90 should be of a material transparent to both light and ultrasounds; as already said, a suitable material is PMMA [poly(methylmethacrylate)].

According to this embodiment, the other device 90 may be disposable and the mechanic device 97 may be permanent as it does not contact the eye, in particular the cornea at all.

In the embodiment of Fig. 3, the distance between the front surface of the mechanic device and the front surface of the cornea is typically in the range of 0.5-2.5 mm. A layer of a viscous transparent coupling agent should be between the eye and the mechanic device.

In the embodiment of Fig. 4, the distance between the front surface of the mechanic device and the front surface of the cornea is typically in the range of 0.5-2.5 mm, while the distance between the front surface of the other device and the front surface of the cornea is typically in the range of 0.5-2.5 mm. A layer of a viscous transparent coupling agent should be between the eye and the mechanic and other devices.

In the embodiment of Fig. 6, the distance between the front surface of the mechanic device and the front surface of the cornea is typically in the range of 5-20 mm, while the distance between the front surface of the other device and the front surface of the cornea is typically in the range of 0.5-2.5 mm. A layer of a viscous transparent coupling agent should be between the eye and the other device.

In the embodiment of Fig. 8, the distance between the front surface of the mechanic device and the front surface of the cornea is typically in the range of 0.5-2.5 mm, while the distance between the front surface of the other device and the front surface of the cornea is typically in the range of 0.5-2.5 mm. A layer of a viscous transparent coupling agent should be between the eye and the mechanic and other devices.

In the embodiment of Fig. 9, the distance between the front surface of the mechanic device and the front surface of the cornea is typically in the range of 5-20 mm, while the distance between the front surface of the other device and the front surface of the cornea is typically in the range of 0.5-2.5 mm. A layer of a viscous transparent coupling agent should be between the eye and the other device.

In the embodiments of Fig. 6 and Fig. 9, the front surface of the mechanic device is in contact with a rear surface of the other device.

In all these embodiments, the electronic devices are at the front surface of the mechanic device.

Elements corresponding in the embodiments of the figures from Fig.4 to Fig.9 are:
- mechanic device (i.e. support device) : 30, 40, 60, 87, 97
- front surface of mechanic device : 31, 41, 68, 88, 98
- other device (e.g. optical device) : (not in Fig.3), 47, 67, 80, 90
- front surface of other device : (not in Fig.3), 48, 61, 81, 91
- electronic devices : 35, 45, 65, 85, 95
- circuitry : 36, 46, 66, 86, 96
- fixation target : 39, 49, 69, 89, 99

The equipments just described allow to implement the general method of the present invention as set out at the beginning of the detailed description.

These equipments allow to detect contemporaneously or almost contemporaneously a plurality of cross-sectional areas (for example areas A1, A2, A3, A4, A5, A6, A7, A8 in Fig.1B) so that a single imaging process covers an angle of at least 90°, more preferably at least 180°; even more preferably 360°; typically, in case of non contemporaneity, the maximum delay between two successive detections of two distinct groups of devices (for example the groups G1, G2, G3, G4, G5, G6, G7, G8 in Fig.1B) may be in the range from 10 mS to 100 mS, more preferably from 10 mS to 40 mS. The various detecting actions are typically independent from each other.

The equipments of Fig.4, Fig.6, Fig.8, Fig.9 and the mentioned variant of the equipment of Fig.4, comprise both an ultrasound assembly and an optical assembly (embodied in two distinct devices or in a single device); therefore, they provide not only ultrasound imaging but also optical observation of an iridocorneal annular zone of an eye. Advantageously, ultrasound imaging and optical observation are carried out contemporaneously or almost contemporaneously; typically, in case of non contemporaneity, the maximum delay between the two kinds of observations may be in the range from 1 S or 10 S.

Alternatively, the other device of the equipments of Fig.4, Fig.6, Fig.8, Fig.9 may be only a support and/or cover for the mechanic device.

## Claims

1. Equipment for ultrasound imaging a plurality of cross-sectional areas (A1, A2, A3, A4, A5, A6, A7, A8) of a peripheral annular volume of an eye (100) comprising:
- a mechanic device (30, 40, 60, 87, 97) having a front surface (31, 41, 68, 88, 98) designed to be located close to a front surface (111) of a cornea (110) of the eye (100), and
- a plurality of ultrasound emitting and receiving electronic devices (35, 45, 65, 85, 95) associated with said mechanic device (30, 40, 60, 87, 97), arranged in an annular manner so as to be distributed around the eye (100) when said mechanic device (30, 40, 60, 87, 97) is located close to the front surface (111) of the cornea (110) of the eye (100), each of said electronic devices being directed toward a cross-sectional area (A1, A2, A3, A4, A5, A6, A7, A8) of the peripheral annular volume of the eye (100) opposite to the device with respect to the eye axis;
wherein said mechanic device (30, 40, 60, 87, 97) is designed to be stationary with respect to the eye (100);
wherein said electronic devices (35, 45, 65, 85, 95) are designed to be stationary with respect to the eye (100).

2. Equipment according to claim 1, further comprising another device (47, 67, 80, 90), wherein said another device (47, 67, 80, 90) and said mechanic device (30, 40, 60, 87, 97) are fixed to each other, and wherein said electronic devices (35, 45, 65, 85, 95) are fixed to said mechanic device (30, 40, 60, 87, 97).

3. Equipment according to claim 2, wherein said another device (67, 90) is disposable and said mechanic device (60, 97) is permanent.

4. Equipment according to claim 2 or 3, wherein the front surface (68, 98) of said mechanic device (60, 90) is located behind a front surface (61, 91) of said another device (67, 90).

5. Equipment according to claim 2 or 3 or 4, wherein said another device (47, 67, 80, 90) is an optical device for the optical observation of an iridocorneal annular zone of the eye (100).

6. Equipment according to claim 1 or 2, wherein said mechanic device (30) is also an optical device for the optical observation of an iridocorneal annular zone of the eye (100).

7. Equipment according to any of the preceding claims, wherein said mechanic device (30, 40, 60, 87, 97) has the shape of a cylinder or a prism or a truncated cone or truncated pyramid, or is annular.

8. Equipment according to any of the preceding claims, wherein said electronic devices (35, 45, 65, 85, 95) are cMUT devices.

9. Equipment according to any of the preceding claims, wherein each of said plurality of cross-sectional areas (A1, A2, A3, A4, A5, A6, A7, A8) is detected through a distinct group of electronic devices (G1, G2, G3, G4, G5, G6, G7, G8).

10. Equipment according to any of the preceding claims, comprising a fixation target (39, 49, 69, 89, 99) for maintaining the eye (100) in a fixed and predetermined position during ultrasound imaging.

11. Equipment according to any of the preceding claims, wherein said electronic devices (35, 45, 65, 85, 95) are arranged to treat tissues in the peripheral annular volume of the eye (100) by radiating them with ultrasounds.

12. Method of ultrasound imaging a peripheral annular volume of an eye (100) through a mechanic device (30, 40, 87) having a concave front surface (31, 41, 88) and a plurality of ultrasound emitting and receiving electronic devices (35, 45, 85) associated with said mechanic device (30, 40, 87) and arranged in an annular manner, the method comprising the steps of :
A) applying a viscous transparent coupling agent to a front surface (111) of a cornea (110) of the eye (100) and/or to the concave front surface (31, 41, 88) of the mechanic device (30, 40, 87),
B) positioning the mechanic device (30, 40, 87) so that the front surface (31, 41, 88) of the mechanic device (30, 40, 87) is close to the front surface (111) of the eye (100) and so that said electronic devices (35, 45, 85) are distributed around the eye (100) and each of said electronic devices is directed toward a cross-sectional area (A1, A2, A3, A4, A5, A6, A7, A8) of the peripheral annular volume of the eye (100) opposite to the device with respect to the eye axis,
C) detecting a plurality of cross-sectional areas (A1, A2, A3, A4, A5, A6, A7, A8) of the peripheral annular volume of the eye (100) through said plurality of ultrasound emitting and receiving electronic devices (35, 45, 85; G1, G2, G3, G4, G5, G6, G7, G8); wherein during step C said mechanic device (30, 40, 87) and said electronic devices (35, 45, 85) are maintained stationary with respect to the eye (100).

13. Method according to claim 12, wherein the plurality of cross-sectional areas (A1, A2, A3, A4, A5, A6, A7, A8) are detected contemporaneously or almost contemporaneously.

14. Method according to claim 12 or 13, wherein said mechanic device (30) is transparent to light and is also used as an optical device for the optical observation of an iridocorneal annular zone of the eye (100), and wherein ultrasound imaging and optical observation are carried out contemporaneously or almost contemporaneously.

15. Method of ultrasound imaging a peripheral annular volume of an eye (100) through a mechanic device (60, 97) having a front surface (68, 98), another device (67, 90) having a concave front surface (61, 91) and being transparent at least to ultrasounds, and a plurality of ultrasound emitting and receiving electronic devices (65, 95) associated with said mechanic device (60, 97) and arranged in an annular manner, the method comprising the steps of :
A) applying a viscous transparent coupling agent to a front surface (111) of a cornea (110) of the eye (100) and/or to the concave front surface (61, 91) of the other device (67, 90),
B) positioning the mechanic device (60, 97) and the other device (67, 90) so that their front surfaces (61, 68, 91, 98) are close to the front surface (111) of the eye (100) and so that said electronic devices (35, 45, 85) are distributed around the eye (100) and each of said electronic devices is directed toward a cross-sectional area (A1, A2, A3, A4, A5, A6, A7, A8) of the peripheral annular volume of the eye (100) opposite to the device with respect to the eye axis,
C) detecting a plurality of cross-sectional areas (A1, A2, A3, A4, A5, A6, A7, A8) of the peripheral annular volume of the eye (100) through said plurality of ultrasound emitting and receiving electronic devices (65, 95; G1, G2, G3, G4, G5, G6, G7, G8); wherein during step C said mechanic device (60, 97) and said another device (67, 90) and said electronic devices (65, 95) are maintained stationary with respect to the eye (100).

16. Method according to claim 15, wherein the plurality of cross-sectional areas (A1, A2, A3, A4, A5, A6, A7, A8) are detected contemporaneously or almost contemporaneously.

17. Method according to claim 15 or 16, comprising further the step of disposing said another device (67, 90) after step C.

18. Method according to claim 15 or 16 or 17, wherein said another device (67, 90) is transparent also to light and is also used for the optical observation of an iridocorneal annular zone of the eye (100), and wherein ultrasound imaging and optical observation are carried out contemporaneously or almost contemporaneously.

## Patentansprüche

1. Ausrüstung zur Ultraschallbildgebung einer Vielzahl von Querschnittsflächen (A1, A2, A3, A4, A5, A6, A7, A8) eines peripheren Ringvolumens eines Auges (100), umfassend:
- eine mechanische Vorrichtung (30, 40, 60, 87, 97) aufweisend eine Vorderfläche (31, 41, 68, 88, 98), die ausgelegt ist, um nah an einer Vorderfläche (111) einer Hornhaut (110) des Auges (100) positioniert zu werden,
und
- eine Vielzahl von ultraschallemittierenden und -empfangenden elektronischen Vorrichtungen (35, 45, 65, 85, 95), die der mechanischen Vorrichtung (30, 40, 60, 87, 97) zugeordnet sind und ringförmig um das Auge (100) herum verteilt anzuordnen sind, wenn die mechanische Vorrichtung (30, 40, 60, 87, 97) nahe der Vorderfläche (111) der Hornhaut (110) des Auges (100) positioniert ist, wobei jede der elektronischen Vorrichtungen auf einen Querschnittsbereich (A1, A2, A3, A4, A5, A6, A7, A8) des peripheren Ringvolumens des Auges (100) gerichtet ist, der sich gegenüber der Vorrichtung in Bezug auf die Augenachse befindet;
wobei die mechanische Vorrichtung (30, 40, 60, 87, 97) so ausgelegt ist, dass sie in Bezug auf das Auge (100) stationär ist;
wobei die elektronischen Vorrichtungen (35, 45, 65, 85, 95) so ausgelegt sind, dass sie in Bezug auf das Auge (100) stationär sind;

2. Ausrüstung nach Anspruch 1, ferner umfassend eine andere Vorrichtung (47, 67, 80, 90), wobei die andere Vorrichtung (47, 67, 80, 90) und die mechanische Vorrichtung (30, 40, 60, 87, 97) miteinander befestigt sind, und wobei die elektronischen Vorrichtungen (35, 45, 65, 85, 95) an der mechanischen Vorrichtung (30, 40, 60, 87, 97) befestigt sind.

3. Ausrüstung nach Anspruch 2, wobei die andere Vorrichtung (67, 90) wegwerfbar ist und die mechanische Vorrichtung (60, 97) dauerhaft ist.

4. Ausrüstung nach Anspruch 2 oder 3, wobei die Vorderfläche (68, 98) der mechanischen Vorrichtung (60, 90) hinter einer Vorderfläche (61, 91) der anderen Vorrichtung (67, 90) positioniert ist.

5. Ausrüstung nach Anspruch 2 oder 3 oder 4, wobei die andere Vorrichtung (47, 67, 80, 90) eine optische Vorrichtung zur optischen Beobachtung einer iridokornealen Ringzone des Auges (100) ist.

6. Ausrüstung nach Anspruch 1 oder 2, wobei die mechanische Vorrichtung (30) auch eine optische Vorrichtung zur optischen Beobachtung einer iridokornealen Ringzone des Auges (100) ist.

7. Ausrüstung nach irgendeinem der vorhergehenden Ansprüche, wobei die mechanische Vorrichtung (30, 40, 60, 87, 97) die Form eines Zylinders oder eines Prismas oder eines Kegelstumpfes oder eines Pyramidenstumpfes hat bzw. ringförmig ist.

8. Ausrüstung nach irgendeinem der vorhergehenden Ansprüche, wobei die elektronischen Vorrichtungen (35, 45, 65, 85, 95) cMUT-Vorrichtungen sind.

9. Ausrüstung nach irgendeinem der vorhergehenden Ansprüche, wobei jeder der Vielzahl von Querschnittsbereichen (A1, A2, A3, A4, A5, A6, A7, A8) durch eine bestimmte Gruppe von elektronischen Vorrichtungen (G1, G2, G3, G4, G5, G6, G7, G8) erfasst wird.

10. Ausrüstung nach irgendeinem der vorhergehenden Ansprüche, umfassend ein Fixierungsziel (39, 49, 69, 89, 99), um das Auge (100) in einer festen und vorbestimmten Position während der Ultraschallbildgebung zu halten.

11. Ausrüstung nach irgendeinem der vorhergehenden Ansprüche, wobei die elektronischen Vorrichtungen (35, 45, 65, 85, 95) angeordnet sind, um Gewebe in dem peripheren Ringvolumen des Auges (100) durch Bestrahlen derselben mit Ultraschall zu behandeln.

12. Verfahren zur Ultraschallbildgebung eines peripheren Ringvolumens eines Auges (100) durch eine mechanische Vorrichtung (30, 40, 87) mit einer konkaven Vorderfläche (31, 41, 88) und einer Vielzahl von ultraschallemittierenden und -empfangenden elektronischen Vorrichtungen (35, 45, 85), die der mechanischen Vorrichtung (30, 40, 87) zugeordnet und ringförmig angeordnet sind, wobei das Verfahren folgende Schritte umfasst:
A) Aufbringen eines viskosen transparenten Haftvermittlers auf eine Vorderfläche (111) einer Hornhaut (110) des Auges (100) und/oder auf die konkave Vorderfläche (31, 41, 88) der mechanischen Vorrichtung (30, 40, 87),
B) Positionieren der mechanischen Vorrichtung (30, 40, 87) so, dass die Vorderfläche (31, 41, 88) der mechanischen Vorrichtung (30, 40 "87) nahe der Vorderfläche (111) des Auges (100) liegt. und so
dass die elektronischen Vorrichtungen (35, 45, 85) um das Auge (100) herum verteilt sind und jede der elektronischen Vorrichtungen
auf einen Querschnittsbereich (A1, A2, A3, A4, A5, A6, A7, A8) des
peripheren Ringvolumens des Auges (100) gerichtet ist, der sich gegenüber der Vorrichtung in Bezug auf die
Augenachse befindet,
C) Erfassen einer Vielzahl von Querschnittsflächen (A1, A2, A3, A4, A5, A6, A7, A8) des peripheren Ringvolumens des Auges (100) durch die Vielzahl von ultraschallemittierenden und -empfangenden elektronischen Geräten (35, 45, 85, G1, G2, G3, G4, G5, G6, G7, G8); wobei während Schritt C die mechanische Vorrichtung (30, 40, 87) und die elektronischen Vorrichtungen (35, 45, 85) in Bezug auf das Auge (100) stationär gehalten werden.

13. Verfahren nach Anspruch 12, wobei die Vielzahl von Querschnittsflächen (A1, A2, A3, A4, A5, A6, A7, A8) gleichzeitig oder fast gleichzeitig erfasst werden.

14. Verfahren nach Anspruch 12 oder 13, wobei die mechanische Vorrichtung (30) für Licht transparent ist und auch als optische Vorrichtung zur optischen Beobachtung einer iridokornealen Ringzone des Auges (100) verwendet wird, und wobei Ultraschallbildgebung und optische Beobachtung zeitgleich oder fast zeitgleich durchgeführt werden.

15. Verfahren zur Ultraschallbildgebung eines peripheren Ringvolumens eines Auges (100) durch eine mechanische Vorrichtung (60, 97), die eine Vorderfläche (68, 98) aufweist; eine andere Vorrichtung (67, 90), die eine konkave Vorderfläche (61, 91) aufweist und zumindest für Ultraschall transparent ist; und eine Vielzahl von ultraschallemittierenden und -empfangenden elektronischen Vorrichtungen (65, 95), die der mechanischen Vorrichtung (60, 97) zugeordnet und ringförmig angeordnet sind, wobei das Verfahren folgende Schritte umfasst:
A) Aufbringen eines viskosen transparenten Haftvermittlers auf eine Vorderfläche (111) einer Hornhaut (110) des Auges (100) und/oder auf die konkave Vorderfläche (61, 91) der anderen Vorrichtung (67, 90),
B) Positionieren der mechanischen Vorrichtung (60, 97) und der anderen Vorrichtung (67, 90) so, dass ihre Vorderflächen (61, 68, 91, 98) nahe der Vorderfläche (111) des Auges (100) liegt. und so
dass die elektronischen Vorrichtungen (35, 45, 85) um das Auge (100) herum verteilt sind und jede der elektronischen Vorrichtungen
auf einen Querschnittsbereich (A1, A2, A3, A4, A5, A6, A7, A8) des peripheren Ringvolumens des Auges (100) gerichtet ist, der gegenüber der Vorrichtung in Bezug auf die Augenachse liegt;
C) Erfassen einer Vielzahl von Querschnittsflächen (A1, A2, A3, A4, A5, A6, A7, A8) des peripheren Ringvolumens des Auges (100) durch die Vielzahl von ultraschallemittierenden und -empfangenden elektronischen Vorrichtungen (65, 95, G1, G2, G3, G4, G5, G6, G7, G8); wobei während Schritt C die mechanische Vorrichtung (60, 97) und die andere Vorrichtung (67,90) und die elektronischen Vorrichtungen (65, 95) in Bezug auf das Auge (100) stationär gehalten werden.

16. Verfahren nach Anspruch 15, wobei die Vielzahl von Querschnittsflächen (A1, A2, A3, A4, A5, A6, A7, A8) gleichzeitig oder fast gleichzeitig erfasst werden.

17. Verfahren nach Anspruch 15 oder 16, ferner umfassend den Schritt des Entsorgens der anderen Vorrichtung (67, 90) nach Schritt C.

18. Verfahren nach Anspruch 15 oder 16 oder 17, wobei die andere Vorrichtung (67, 90) auch für Licht transparent ist und auch zur optischen Beobachtung eines iridokornealen Ringbereiches des Auges (100) verwendet wird, und wobei Ultraschallbildgebung und optische Beobachtung zeitgleich oder fast zeitgleich durchgeführt werden.

## Revendications

1. Équipement d'imagerie par ultrasons d'une pluralité de sections transversales (A1, A2, A3, A4, A5, A6, A7, A8) d'un volume annulaire périphérique d'un œil (100) comprenant :
- un dispositif mécanique (30, 40, 60, 87, 97) ayant une surface avant (31, 41, 68, 88, 98) conçu pour être situé à proximité de la surface avant (111) d'une cornée (110) de l'œil (100) ;
et
- une pluralité de dispositifs émetteurs et récepteurs d'ultrasons (35, 45, 65, 85, 95), associés au dit dispositif mécanique (30, 40, 60, 87, 97), disposés de façon annulaire, de sorte à
être répartis autour de l'œil (100) lorsque lesdits dispositif mécanique (30, 40, 60, 87, 97) est situé à proximité de la surface avant (111) de la cornée (110) de l'œil (100), chacun des dits dispositifs électroniques étant orientés vers une zone transversale (A1, A2, A3, A4, A5, A6, A7, A8) du volume annulaire périphérique de l'œil (100) opposée au dispositif par rapport à l'axe de l'œil ;
dans lequel ledit dispositif mécanique (30, 40, 60, 87, 97) est conçu pour être statique par rapport à l'œil (100) ;
dans lequel lesdits dispositifs électroniques (35, 45, 65, 85, 95) sont conçus pour être statiques par rapport à l'œil (100).

2. Équipement selon la revendication 1, comprenant en outre un autre dispositif (47, 67, 80, 90), dans lequel ledit autre dispositif (47, 67, 80, 90) et ledit dispositif mécanique (30, 40, 60, 87, 97) sont fixés l'un à l'autre, et dans lequel lesdits dispositifs électroniques (35, 45, 65, 85, 95) sont fixés au dit dispositif mécanique (30, 40, 60, 87, 97).

3. Équipement selon la revendication 2, dans lequel ledit autre dispositif (67, 90) est à usage unique et ledit dispositif mécanique (60, 97) est à usage permanent.

4. Équipement selon la revendication 2 ou 3, dans lequel a surface avant (68, 98) du dit dispositif mécanique (60, 90) se trouve derrière une surface avant (61, 91) du dit autre dispositif (67, 90).

5. Équipement selon la revendication 2 ou 3 ou 4, dans lequel ledit autre dispositif (47, 67, 80, 90) est un dispositif optique destiné à l'observation optique d'une zone irrido-cornéenne annulaire de l'œil (100).

6. Équipement selon la revendication 1 ou 2, dans lequel ledit dispositif mécanique (30) est un dispositif optique destiné à l'observation optique d'une zone irrido-cornéenne annulaire de l'œil (100).

7. Équipement selon l'une quelconque des revendications précédentes, dans lequel ledit dispositif mécanique (30, 40, 60, 87, 97) a la forme d'un cylindre ou d'un prisme ou d'un cône tronqué ou d'une pyramide tronquée ou est annulaire.

8. Équipement selon l'une quelconque des revendications précédentes, dans lequel lesdits dispositifs électroniques (35, 45, 65, 85, 95) sont des dispositifs de type cMUT.

9. Équipement selon l'une quelconque des revendications précédentes, dans lequel chacune de ladite pluralité de zones transversales (A1, A2, A3, A4, A5, A6, A7, A8) est détectée au travers d'un groupe distinct de dispositifs électroniques (G1, G2, G3, G4, G5, G6, G7, G8).

10. Équipement selon l'une quelconque des revendications précédentes, comprenant un élément de fixation (39, 49, 69, 89, 99) pour maintenir l'œil (100) dans une position fixe et prédéterminée pendant le processus d'imagerie à ultrasons.

11. Équipement selon l'une quelconque des revendications précédentes, dans lequel lesdits dispositifs électroniques (35, 45, 65, 85, 95) sont disposés pour traiter les tissus du volume annulaire périphérique de l'œil (100) par irradiation à l'aide des ultrasons.

12. Procédé d'imagerie par ultrasons d'un volume annulaire périphérique d'un œil (100) au moyen d'un dispositif mécanique (30, 40, 87) ayant une surface avant concave (31, 41, 88) et une pluralité de dispositifs électroniques émetteurs et récepteurs d'ultrasons (35, 45, 85) associés au dit dispositif mécanique (30, 40, 87) et disposés de façon annulaire, comprenant les étapes consistant à :
A) appliquer un agent de couplage visqueux transparent sur une surface avant (111) de la cornée (110) de l'œil (100) et/ou à la surface avant (31, 41, 88) concave du dispositif mécanique (30, 40, 87) ;
B) positionner le dispositif mécanique (30, 40, 87) de sorte que la surface avant (31, 41, 88) du dispositif mécanique (30, 40, 87) est proche
de la surface avant (111) de l'œil (100) et de
sorte que lesdits dispositifs électroniques (35, 45, 85) sont répartis autour de l'œil (100) et chacun des dits dispositifs électroniques est
orienté vers une zone transversale (A1, A2, A3, A4, A5, A6, A7, A8) du volume annulaire périphérique de l' œil (100) opposée au dispositif par rapport à l'axe de l'œil ;
C) détecter une pluralité de zones transversales (A1, A2, A3, A4, A5, A6, A7, A8) du volume annulaire périphérique de l'œil (100) à l'aide de ladite pluralité de dispositifs électroniques (35, 45, 85; G1, G2, G3, G4, G5, G6, G7, G8) émetteurs et récepteurs d'ultrasons ; dans lequel à l'étape C, ledit dispositif mécanique (30, 40, 87) et les dits dispositifs électroniques (35, 45, 85) sont maintenus en position statique par rapport à l'œil (100) .

13. Procédé selon la revendication 12, dans lequel la pluralité de zones transversales (A1, A2, A3, A4, A5, A6, A7, A8) sont détectées de façon simultanée ou quasi simultanée.

14. Procédé selon la revendication 12 ou 13, dans lequel ledit dispositif mécanique (30) est transparent et également utilisé comme dispositif optique pour l'observation optique d'une zone irrido-cornéenne annulaire de l'œil (100), et dans lequel l'imagerie par ultrason et l'observation optique sont effectuées de façon simultanée ou quasi simultanée.

15. Procédé d'imagerie par ultrason d'un volume annulaire périphérique d'un œil (100) au moyen d'un dispositif mécanique (60, 97) présentant une surface avant (68, 98), un autre dispositif (67, 90) ayant une surface avant concave (61, 91) et étant transparent au moins aux ultrasons, et une pluralité de dispositifs électroniques émetteurs et récepteurs d'ultrasons (65, 95) associés au dit dispositif mécanique (60, 97) et disposés de façon annulaire, la méthode comprenant les étapes consistant à :
A) appliquer un agent de couplage visqueux transparent sur une surface avant (111) de la cornée (110) de l'œil (100) et/ou sur la surface avant (61, 91) concave de l'autre dispositif (67, 90) ;
B) positionner le dispositif mécanique (60, 97) et l'autre dispositif (67, 90) de sorte que leurs surfaces avant (61, 68, 91, 98) soient proches
de la surface avant (111) de l'œil (100) et de
sorte que lesdits dispositifs électroniques (35, 45, 85) sont répartis autour de l'œil (100) et chacun des dits dispositifs électroniques est
orienté vers une zone transversale (A1, A2, A3, A4, A5, A6, A7, A8) du volume annulaire périphérique de l' œil (100) opposée au dispositif par rapport à l'axe de l'œil ;
C) détecter une pluralité de zones transversales (A1, A2, A3, A4, A5, A6, A7, A8) du volume annulaire périphérique de l'œil (100) à l'aide de ladite pluralité de dispositifs électroniques (65, 95; G1, G2, G3, G4, G5, G6, G7, G8) émetteurs et récepteurs d'ultrasons ; dans lequel à l'étape C, ledit dispositif mécanique (60, 97) et ledit autre dispositif (67, 90) et les dits dispositifs électroniques (65, 95) sont maintenus en position statique par rapport à l' œil (100).

16. Procédé selon la revendication 15, dans lequel la pluralité de zones transversales (A1, A2, A3, A4, A5, A6, A7, A8) sont détectées de façon simultanée ou quasi simultanée.

17. Procédé selon la revendication 15 ou 16, comprenant en outre l'étape d'élimination dudit autre dispositif (67, 90) après l'étape C.

18. Procédé selon la revendication 15, 16 ou 17, dans lequel ledit autre dispositif (67, 90) est également transparent et également utilisé pour l'observation optique d'une zone irrido-cornéenne annulaire de l'œil (100), et dans lequel l'imagerie par ultrason et l'observation optique sont effectuées de façon simultanée ou quasi simultanée.
